# EUROPEAN PATENT APPLICATION

(11) **EP 2 226 015 A1**
(43) Date of publication of application: **08.09.2010**
(21) Application number: 10155847.6
(22) Date of filing: 08.03.2010
(51) Int. Cl.: A61B 17/00

(54) **Haemorrhoidopexy device**

(30) Priority: 07.03.2009 GB 0903997
(71) Applicant: Morsey, Hesham, London N3 3RU (GB)
(72) Inventor: Morsey, Hesham, London N3 3RU (GB)
(74) Representative: Williams, Paul Edwin

(57) **Abstract**

A transparent plastic device to assist in the application of a purse string stitch during a haemorrhoidopexy operation including complete full thickness circular cuts 2 at 2 and 4 cm from the base, curved bridges 3 spanning the cuts and circular holes at the base 4 to allow the device to be secured in place with stitches.

## Description

This invention relates to a device to be used in haemorrhoidopexy operation.

Treatment of haemorhoids using circular stapler is two steps procedure. First step is the application of circular purse-string stitch inside the anal canal and the second step is using the circular stapler to remove a circular ano-rectal tissue to complete the procedure.

The application of purse-string stitch is the most crucial part of the operation. The current technique used requires several applications of anoscope in order to complete the circular purse-string stitch, this will result in poor visualization in the majority of patients, especially in males with narrow pelvis and when mucosal bleeding results from needle application. The end result is usually unsatisfactory application of purse-string, as the stitches will not be in the same line, because of the need for several re-application of the anoscope with each stitch. This will result in incomplete excision, continuation of patients symptoms, patient's dissatisfaction, and in the majority of cases further management is required. The increase in the recurrence rate has been linked to cases in which doughnuts are deficient as a result of incomplete excision.

The new invented device will make the procedure, easier, faster and more efficient with reduced recurrence rate. The new invented device is an anal transparent plastic material, so that the surgeon can identify clearly where to apply the purse-string in relation to the anal sphincter. A complete full thickness circular cut, 2 cm in width, is made around the device at 2 and 4 cm from the base in order to be able to perform a complete purse-string stitch with single application of the device. Curved bridges on the inside of the device at 12,6,9,3 O'clock are made across the circular hole. This will allow passing the needle behind the bridges during the stitching process and being able to remove the device on completion leaving the purse string stitch behind, ready to apply the circular stapler to complete the procedure. Circular holes at the base of the device, so it can be fixed to the skin around the anus with stitches.

The invention will now be described solely by way of example and with reference to the accompanying drawings in which:
- Figure 1: shows sagital section of the device (the inside of the device), complete circular cut, curved bridges across the circular cut, circular holes at the base for fixation of the device.
- Figure 2: shows the out side appearance of the device.

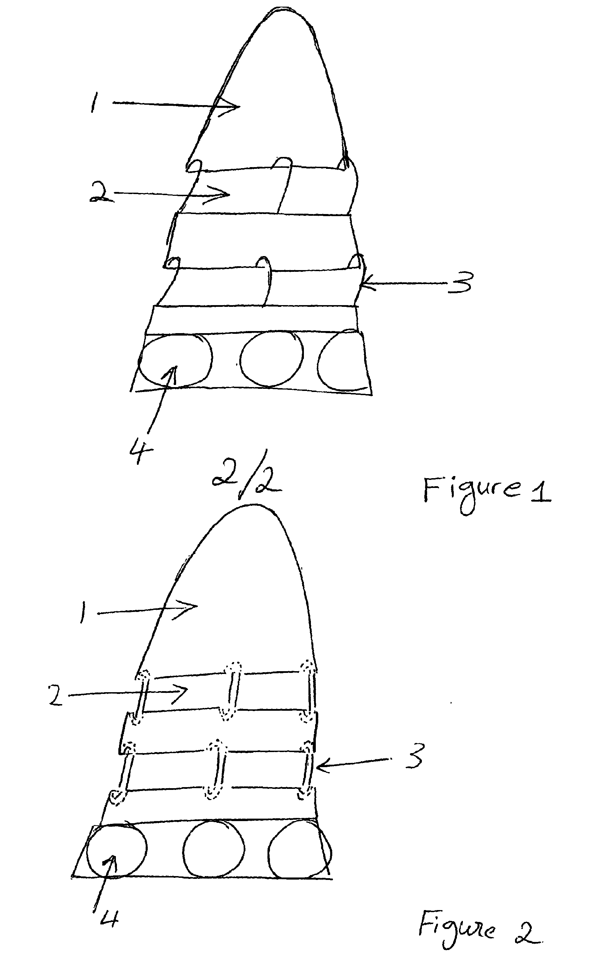

In figure 1, a transparent plastic device from inside 1, circular cut in the device2, Curved bridges across the circular cut 3, circular holes at the base of the device 4.

In figure 2, a transparent plastic device 1, circular cut in the device 2, bridges 3, circular holes at the base.

## Claims

1. Anal transparent plastic device has complete full thickness circular cuts at 2 and 4 cm from the base of the device.

2. Anal transparent plastic device according to claim1, in which the device has curved bridges across the circular cuts at 12,6,3,9 O'clock.

3. Anal transparent plastic device according to claim1, in which the device's base has holes for fixation to the skin around the anus using stitches.

4. Anal transparent plastic device according to claim1, Single application of the device to perform purse-string stitch at 2 or 4 cm from the base as appropriate.
